## Europäisches Patentamt

## European Patent Office

## Office européen des brevets

(19)

(11) Veröffentlichungsnummer: **0 064 281**

**B1**

(12) **EUROPÄISCHE PATENTSCHRIFT**

(45) Veröffentlichungstag der Patentschrift:
**08.04.87**

(51) Int. Cl.⁴: **A 61 B 5/02,** G 05 D 7/01

(21) Anmeldenummer: **82103679.5**

(22) Anmeldetag: **29.04.82**

(54) Ablassventil für ein Druckmedium aus einem Blutdruckmessgerät.

(30) Priorität: **04.05.81 DE 3117546**

(43) Veröffentlichungstag der Anmeldung:
**10.11.82 Patentblatt 82/45**

(45) Bekanntmachung des Hinweises auf die Patenterteilung:
**08.04.87 Patentblatt 87/15**

(84) Benannte Vertragsstaaten:
**AT BE CH DE FR GB IT LI NL SE**

(56) Entgegenhaltungen:
**CH-A-619 362**
**DE-A-2 759 119**
**DE-A-2 826 201**
**GB-A-1 181 405**
**US-A-3 557 833**
**US-A-3 779 236**

(73) Patentinhaber: **Richard Kallmeyer & Co., Im Farchet 15 Postfach 1320, D-8170 Bad Tölz (DE)**

(72) Erfinder: **Stiebler, Franz, Am Ried 8, D-8170 Bad Tölz (DE)**

(74) Vertreter: **Schwabe, Hans- Georg, Dipl.- Ing., Patentanwälte Schwabe, Sandmair, Marx Stuntzstrasse 16, D-8000 München 80 (DE)**

LIBER, STOCKHOLM 1987

## Beschreibung

Die Erfindung betrifft ein Ablaßventil nach dem Oberbegriff von Anspruch 1 bzw. Anspruch 2, aus einem Blutdruckmeßgerät, mit einer Kammer, deren Innenraum mit der Zuströmseite des Druckmediums verbunden ist, mit einem, zumindest teilweise in der Kammer befindlichen, Hohlteil mit elastischen Wandungen, das rohrförmig, an einem Ende offen und mit einem Innenraum mit im wesentlichen gleichförmigem Querschnitt ausgebildet ist, wobei in mindestens einer der in der Kammer befindlichen Wandungen des Hohlteils ein Schlitz vorgesehen ist und die Kammer im übrigen dichtend geschlossen ist und wobei der Innenraum des Hohlteils mit der Abströmseite des Druckmediums verbunden ist, und mit einem die Öffnung des Schlitzes beeinflussenden Element.

Ein derartiges Ablaßventil ist aus der DE-OS 27 59 119 bekannt.

Das Druckmedium, üblicherweise Luft, wird bei den Blutdruckmeßgeräten nach Riva-Ricci in eine um den Oberarm des Patienten gelegte Manschette gepumpt, bis die Blutzufuhr zum Arm abgeschnürt ist. Dann wird die Luft langsam abgelassen und es wird an einem Manometer beobachtet, bei welchem Drücken typische Strömungsgeräusche, die Korotkoff-Geräusche auftreten und wieder verschwinden.

Dabei ist es, insbesondere bei ungeübten Beobachtern, wichtig, daß der Druck langsam und vor allem mit konstanter Ablaßgeschwindigkeit abnimmt, worunter hier verstanden sein soll, daß die Druckdifferenz pro Zeiteinheit konstant ist. Hierbei sind nach den Vorschriften für die Blutdruckmessung ungefähr 0,27 bis 0,54 kpa (0,2 bis 0,4 "mmHg")/s, speziell 0,4 kpa (0,3 "mmHg")/s vorgesehen.

Dem soll die eingangs genannte Ablaßvorrichtung dienen.

Unter Zuströmseite wird hier die Seite des Ventils im Ablaßweg verstanden, die der Manschette zugekehrt ist, von der also das unter erhöhtem Druck stehende Medium beim Ablassen kommt. Entsprechend ist die Abströmseite zu verstehen.

Nachteilig an der eingangs genannten Ablaßvorrichtung ist jedoch, daß sich mit den in der DE-OS 27 59 119 genannten Elementen, nämlich Drahtstiften oder Haken, die durch den Schlitz gesteckt werden, wodurch dieser in der Umgebung der Stifte leicht geöffnet wird, vielfach keine hinreichend konstante Ablaßgeschwindigkeit einstellt. Ferner ist die Ablaßgeschwindigkeit in vielen Fällen auch bei Verwendung einer Vorrichtung zu ihrer Konstanthaltung von Fall zu Fall verschieden, weil sie vom Volumen der Manschette beeinflußt ist, das (bei jeweils gleichem Druck) wiederum durch unterschiedliches Anlegen der Manschette mitbestimmt wird. Ferner läßt sich bei der bekannten Vorrichtung das Ausströmen von Luft ohne Zusatzeinrichtung nie ganz abstellen.

Aus der US-A-3 779 236 ist bereits ein Ablaßventil für ein Druckmedium aus einem Blutdruckmeßgerät bekannt, bei welchem die Ablaßgeschwindigkeit durch den Benutzer des Geräts bestimmt werden kann. Dieses Ablaßventil weist eine Kammer auf, in welcher sich ein Hohlteil mit elastischen Wandungen befindet, das eine Ablaßöffnung zwischen der Kammer und der Außenseite des Ventils dichtend abschließt. Ein Element beeinflußt die Abdichtung der Öffnung durch das Hohlteil und bewirkt dann das Ablassen des Druckmediums, wenn es in radialer Richtung leicht eingedrückt wird und demzufolge das Hohlteil so verformt, daß ein Ablaßweg zwischen den Wandungen des Hohlteils und der Ablaßöffnung entsteht. Um eine kontinuierlich bestimmbare Einstellung der Ablaßgeschwindigkeit zu gewährleisten, ist nach der US-A-3 779 236 vorgesehen, das Element von außen steuerbar verstellbar und als die Außenwandung des elastischen Hohlteils drückend beaufschlagende Einrichtung auszubilden. Nachteilig bei diesem bekannten Gerät ist jedoch, daß nicht automatisch eine konstante Ablaßgeschwindigkeit erzielt werden kann, sondern dies vielmehr dem Geschick des Benutzers überlassen bleibt, indem er die Handtaste mehr oder weniger geschickt betätigt.

Die Erfindung schafft daher ein Ablaßventil der angegebenen Gattung, das es insbesondere erlaubt, auch bei unterschiedlichen Betriebsbedingungen eine stets gleiche und konstante Ablaßgeschwindigkeit zu erzielen.

Dies wird durch die Merkmale des kennzeichnenden Teils des Anspruchs 1 bzw. des Anspruchs 2 erreicht. Das hat insbesondere den Vorteil, daß eine konstante Ablaßgeschwindigkeit in ihrer Größe von der Bedienungsperson eingestellt werden kann und damit den jeweiligen Gegebenheiten und/oder den Gewohnheiten der Bedienungsperson angepaßt werden kann. Insbesondere können dadurch die Funktion eines Sperrventils, eines Konstantventils und eines Schnellablaßventils in einer Ablaßvorrichtung von sehr einfachem Aufbau vereinigt werden.

Diese Ausbildung nach Anspruch 1 hat insbesondere den Vorteil, daß die Vorspannung des Schlitzes und damit die Konstanz der Ablaßgeschwindigkeit sehr genau dosierbar wird und sich der Aufbau des Ablaßventiles insgesamt vereinfacht. Der Abströmkanal stellt bei diesem Aufbau sicher, daß das durch den Schlitz zuströmende, abzulassende Druckmedium aus dem Ventil weiterbefördert werden kann. Bevorzugt ist bei einem Ablaßventil, bei dem der Innenraum des elastischen Hohlteils zylindrisch ausgebildet ist, das Spreizelement ebenfalls zylindrisch und in der Kammer so gelagert, daß es durch Drehung eines aus der Kammer herausragenden Teils axial verschoben wird. Hierdurch wird das Spreizelement an der Kammer bei besonders einfacher Herstellung der Teile besonders sicher gehalten und die Verschiebung des Spreizelementes in axialer Richtung leichter regulierbar.

Bevorzugt sind für den Verschiebungsweg des Einstellelementes Rastpunkte vorgesehen. Dies ist besonders sinnvoll, wenn die Längsverschiebung direkt, also z.B. nicht über eine Drehbewegung, erzeugt wird, wobei z.B. drei Rastpunkte (völliger Verschluß, Ablaß etwa 0,4 kPa/s und Schnellablaß) vorgesehen sein können um die Bedienung weiter zu vereinfachen.

Nachfolgend wird die Erfindung anhand der beigefügten Zeichnungen, auf die wegen ihrer großen Klarheit und Übersichtlichkeit bezüglich der Öffenbarung ausdrücklich verwiesen wird, noch näher beschrieben.

Es zeigen

Fig. 1 eine Ventilkammer gemäß der Erfindung zur Aufnahme eines elastischen Hohlteils, im Längsschnitt;

Fig. 2 eine bevorzugte Ausführungsform eines elastischen Hohlteils im Längsschnitt;

Fig. 3 das Teil der Fig. 2 in Aufsicht;

Fig. 4 eine bevorzugte Ausführungsform eines Einstellelementes gemäß der Erfindung, im Längsschnitt;

Fig. 5 eine vergrößerte Darstellung des Bereichs V aus Fig. 4;

Fig. 6 die Teile aus Fig. 1, 2 und 4 im Zusammenbau; und

Fig. 7 eine weitere Ausführungsform der Erfindung, teilweise im Schnitt.

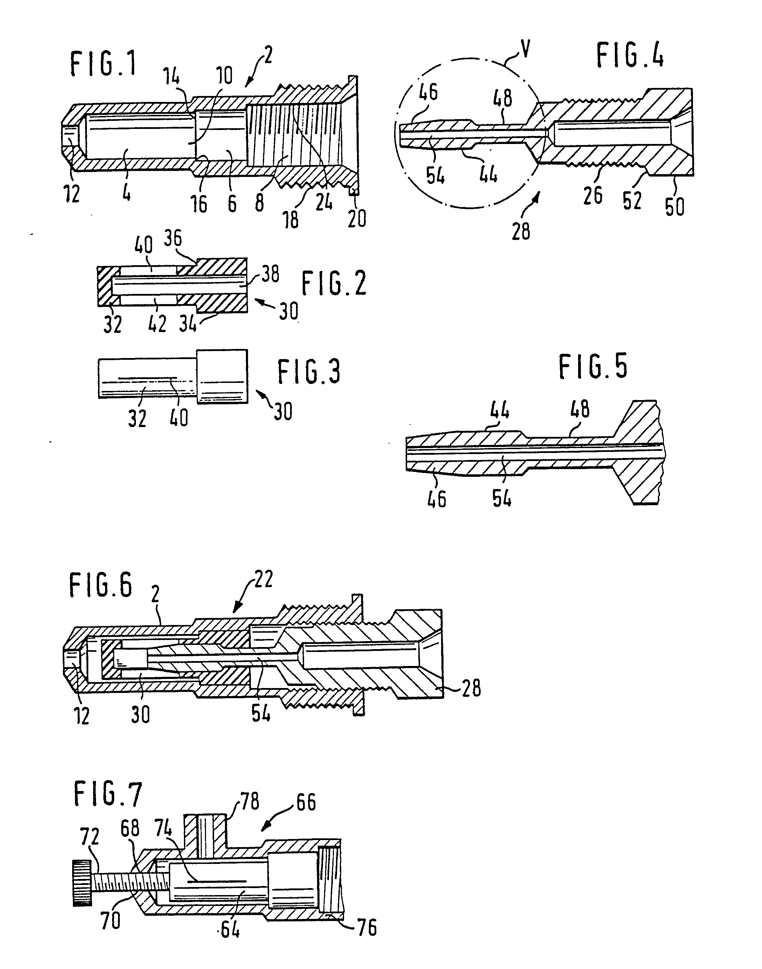

Die Kammer oder der Hauptkörper 2 des Ventils besteht aus mehreren hohlzylindrischen Abschnitten 4, 6 und 8 unterschiedlichen Durchmessers, die ineinander übergehen und zusammen den Innenraum 10 bilden. Der vordere (in der Fig. 1 links) Abschnitt 4 ist vorne bis auf eine Durchlaßöffnung 12 verschlossen. Die Öffnung 12 ist zum Anschluß an die Zuströmseite bestimmt, beispielsweise an einen (nicht gezeigten) Schlauch zur Manschette. Dieser wird zweckmäßig dichtend über das von dem Abschnitt 4 gebildete Rohrstück bis an den durch den etwas größeren außendurchmesser des Abschnitts 6 gebildeten Absatz 14 aufgeschoben.

Der als Abschnitt 6 bezeichnete zylindrische Teil hat wie gesagt einen etwas größeren Außendurchmesser, vor allem aber einen etwas größeren Innendurchmesser, wodurch sich innen ein Absatz 16 bildet.

Außen- und Innendurchmesser des nächsten Abschnitts 8 sind wiederum etwas vergrößert. Allerdings braucht die axiale Lage der jeweiligen inneren und äußeren Stufen der Abschnitte, wie auch aus der Fig. 1 ersichtlich ist, nicht übereinzustimmen.

Abschnitt 8 trägt ein Außengewinde 18, auf das eine (nicht gezeigte) Überwurfmutter aufschraubbar ist, die im Zusammenwirken mit dem Flansch 20 erlaubt, das Ablaßventil 22 (Fig. 6) beispielsweise am Gehäuse oder einer Lasche des Blutdruckmessers (nicht gezeigt) zu befestigen.

Der Abschnitt 8 weist ferner an seinem (in Fig. 1) rechten abströmseitigen Ende ein Innengewinde 24 zur Aufnahme des Außengewindes 26 des Einstellelementes 28 (Fig. 4) auf.

Das Hohlteil mit elastischen Wandungen ist in Fig. 2 als ganz aus Gummi, bevorzugt Naturkautschuk, bestehender, abgestufter Zylinder 30 ausgebildet. Der (in den Figuren) linke längere Abschnitt 32 ist dem Abschnitt 6 der Kammer 2 derart angepaßt, daß er um ein geringes kürzer ist, so daß die Öffnung 12 nicht verschlossen wird, und einen etwas geringeren Durchmesser hat, so daß die Oberfläche des ersten Abschnitts 32 der zuströmenden Luft frei zugänglich ist.

Der zweite Abschnitt 34 hat einen etwas größeren Außendurchmesser (Stufe 36), so daß er gut sitzend in den mittleren Abschnitt 6 der Kammer 2 paßt, dem er auch in der Länge entspricht.

Der Zylinder 30 ist von der Seite des größeren Durchmessers her mit einer zentrischen Sacklochbohrung 38 konstanten Durchmessers versehen, die bis kurz vor das linke Ende des Zylinders 30 führt. Die Bohrung hat einen Durchmesser, der ca. 60 % des kleineren Durchmessers (Abschnitt 32) entspricht.

Im ersten Abschnitt 32 sind in dessen mittleren Bereich über etwa 2/3 der Länge des ersten Abschnitts 32 zwei dünne, achsparallele Schlitze 40 und 42, die von der Außenseite zum SSackloch 38 führen, ausgebildet. Die Schlitze sind mit einer Rasierklinge oder einem so dünnen Instrument ausgeführt, daß sie sich normalerweise durch die Elastizität des Materials des Teils 30 wieder schließen und somit bei unbeeinflußtem Teil 30 verschlossen sind. Die Schlitze sind an diametral gegenüberliegenden Stellen des Mantels des Zylinderabschnitts 32 ausgeführt.

Fig. 3 zeigt noch einmal eine Aufsicht auf das elastische, zylindrische Hohlteil 30.

In Fig. 4 ist das Einstellelement gezeigt. Sein vorderer Bereich besteht im wesentlichen aus einem Spreizzylinder 44, der vorne zum kontinuierlichen Öffnen der Schlitze in einen Konus 46 ausläuft. Dadurch wird der Druckabfall und seine Verstellung gleichmäßiger. Der Konus ermöglicht auch das leichtere Einführen in die Einführöffnung des Sacklochs 38 und verhindert, daß dessen Innenwand bei der Hin- und Herbewegung beschädigt wird.

Über ein Verbindungsstück 48 ist der Spreizzylinder mit einem Einschraubteil 50 versehen. Das Verbindungsstück ist im Durchmesser kleiner als der Zylinder 44 und der Konus 46 gehalten, damit sich der Druck auf das Gummiteil auf den kurzen Bereich 44, 46 beschränkt. Damit kann der Verstellwiderstand klein gehalten werden. Das Einschraubteil 50 weist in einem mittleren Bereich ein zum Innengewinde 24 des Hauptkörpers 2 passendes Außengewinde 26 auf.

Das Ende des Einschraubteils ist verdickt und mit Rändelung zum Angriff durch die Hand ausgebildet. Durch die Verdickung ergibt sich ferner ein Anschlagabsatz 52, der vorzugsweise, wie dargestellt, der Öffnung des Abschnitts 8 entspricht.

Das ganze Einstellelement 28, das im wesentlichen um die Längsachse rotationssymmetrisch ist, ist von einer zentrischen, als Abströmkanal dienenden Axialbohrung 54 durchsetzt, die in Abströmrichtung angeschlossen ist oder deren Ende, wie in Fig. 4 gezeigt, ins Freie mündet. Die Axialbohrung kann, auch aus Gewichtsersparnisgründen, hinten etwas erweitert sein.

Die Kammer und das Einstellelement sind beispielsweise Drehteile aus Messing. Das Gummiteil 30 ist in einer Ausführungsform mit Siliconöl geschmiert, um eine bessere Bewegung des Einstellelementes 28 zu ermöglichen. Bei den verwendeten Materialien (Naturkautschuk) ist es aber auch gut möglich, ohne Schmierung auszukommen. Dies hat den Vorteil, daß diese sich dann auch nicht verlieren kann und der Verstellwiderstand sich nicht ändert.

Fig. 5 zeigt das Spreizteil 44, 46 und das Verbindungsstück 48 noch einmal in größerem Maßstab. In Fig 6 sind die Teile 2, 30 und 28 im Zusammenbau gezeigt. Die Kreuzschraffur soll keine Angabe des Materials sein, sondern die Übersichtlichkeit erhöhen. Befindet sich das Einstellteil 28 in zurückgezogener, herausgedrehter Stellung (wobei im Regelfall der Spreizzylinder sich noch innerhalb des elastischen Teils 30, aber dort im zweiten Abschnitt 34 befindet), so werden die Schlitze überhaupt nicht aufgespreizt und durch den Druck des zuströmenden Mediums nur noch fester geschlossen. (Hingegen wäre es möglich, Luft durch den Kanal 54, die Schlitze 40 und 42 und die Öffnung 12 zur Manschette zu pumpen). Bei einem Hereindrehen des Spreizzylinders 44 spreizt dieser durch seine gegenüber dem Durchmesser der Sacklochbohrung etwas größeren Durchmesser die Schlitze etwas auf. Diese werden durch den Zuströmdruck aus der Manschette mehr oder minder geschlossen, so daß sich eine gleichförmige Ablaßgeschwindigkeit ergibt. Diese läßt sich durch Herein- oder Herausdrehen des Elements 28 sehr gut dosieren. Durch noch weiteres Hereindrehen können die Schlitze so gespreizt werden, daß sich eine Art Schnellablaß ergibt. Eine Beschädigung beispielsweise der Vorderwand innen des Teils 30 vermeidet dabei der Anschlagabsatz 52. Festzuhalten ist, daß sich in jeder Stellung des Spreizzylinders die Manschette aufpumpen läßt.

In Fig. 7 ist eine Ausführungsform gezeigt, bei der die das Gummiteil 64, das entsprechend dem Teil 30 ausgebildet ist, aufnehmende, im wesentlichen zylindrische Kammer 66 an ihrer in Fig. 7 linken Seite eine zentrische, mit einem Gewinde 68 versehene Bohrung 70 aufweist. In das Gewinde ist eine mit einer Rändelung versehene Schraube 72 einschraubbar. Bei genügend tiefem Einschrauben in das Innere der Kammer 66 hinein drückt die Schraube auf die linke vordere Abschlußwandung des Gummiteils 64. Dadurch wird bewirkt, daß sich die Schlitze (nur einer, 74, ist in Fig. 7 sichtbar) noch gleichmäßiger aufspreizen. Am rechten offene Ende des Teiles 66 ist ein Innengewinde 76 zum Einschrauben einer (nicht gezeigten) Halteeinrichtung für das Gummiteil 64 vorgesehen, welche Haltevorrichtung natürlich mit einer Abströmöffnung für das ausströmende Druckmedium versehen sein muß.

Die Zufuhr des zuströmenden Druckmediums erfolgt durch den seitlichen Stutzen 78, auf den ein mit der Manschette des Blutdruckmeßgerätes in Verbindung stehender Schlauch aufsteckbar ist.

## Patentansprüche

1. Ablaßventil für ein Druckmedium aus einem Blutdruckmeßgerät, mit einer Kammer (2), deren Innenraum (10) mit der Zuströmseite (12) des Druckmediums verbunden ist, mit einem, zumindest teilweise in der Kammer befindlichen, Hohlteil (30) mit elastischen Wandungen, das rohrförmig, an einem Ende offen und mit einem Innenraum mit im wesentlichen gleichförmigem Querschnitt ausgebildet ist, wobei in mindestens einer der in der Kammer befindlichen Wandungen des Hohlteils (30) ein Schlitz (40, 42) vorgesehen ist und die Kammer im übrigen dichtend geschlossen ist und wobei der Innenraum (38) des Hohlteils mit der Abströmseite des Druckmediums verbunden (54) ist, und mit einem die Öffnung des Schlitzes beeinflussenden Element (28), dadurch gekennzeichnet, daß das Element als Spreizteil (28) mit einem dem des rohrförmigen Hohlteils (30) entsprechenden, jedoch etwas verdickten Querschnitt (44) ausgebildet und zur axialen Verschiebbarkeit im Innenraum (38) des Hohlteils gelagert (24, 26) ist, und einen zum Innenraum (38) des Hohlteils offenen Abströmkanal (54) aufweist, und daß das Element von außen steuerbar verstellbar ausgebildet ist.

2. Ablaßventil für ein Druckmedium aus einem Blutdruckmeßgerät, mit einer Kammer (66), deren Innenraum mit der Zuströmseite (78) des Druckmediums verbunden ist, mit einem, zumindest teilweise in der Kammer befindlichen Hohlteil (64) mit elastischen Wandungen, das rohrförmig, an einem Ende offen und mit einem Innenraum mit im wesentlichen gleichförmigem Querschnitt ausgebildet ist, wobei in mindestens einer der in der Kammer (66) befindlichen Wandungen des Hohlteils (64) ein Schlitz (74) vorgesehen ist und die Kammer im übrigen dichtend geschlossen ist und wobei der Innenraum des Hohlteils (64) mit der Abströmseite des Druckmediums verbunden ist, und mit einem die Öffnung des Schlitzes beeinflussenden Element (72), dadurch gekennzeichnet, daß das Element durch eine in einem Gewinde (68) in der Wand der Kammer (66) angeordnete Schraube (72) gebildet ist, die auf die vordere Abschlußwand des rohrförmigen Hohlteils (64) drückt, und sich die Schlitze (74) parallel auf den Seitenwandungen des Hohlteils (64) erstrecken, und daß das Element von außen steuerbar verstellbar ausgebildet ist.

3. Ablaßventil nach Anspruch 1, bei dem der Innenraum (38) des elastischen Hohlteils (30) zylindrisch ausgebildet ist, dadurch gekennzeichnet, daß das Spreizelement (28, 44) ebenfalls zylindrisch ist und in der Kammer (2) so gelagert (24, 26) ist, daß es durch Drehung eines aus der Kammer herausragenden Teils (50) axial verschoben wird.

4. Ablaßventil nach einem der vorhergehenden Ansprüche, dadurch gekennzeichnet, daß für den Verschiebungsweg des Elementes (28; 72) Rastpunkte vorgesehen sind.

**Claims**

1. An outlet valve for a pressure fluid from a sphygmomanometer, having a chamber (2), the interior (10) of which is connected to the inflow side (12) of the pressure fluid, having a hollow member (30) with resilient walls, which is at least partially situated in the chamber and is tubular in construction, open at one end and has an interior with a substantially uniform cross-section, wherein a slit (40, 42) is provided in at least one of the walls of the hollow member (30) which are situated in the chamber and the chamber is otherwise closed with a sealing action and wherein the interior (38) of the hollow member is connected to the outflow side of the pressure fluid (54), and having an element (28) influencing the opening of the slit, characterised in that the element is constructed in the form of an expanding member (28) having a cross-section (44) corresponding to that of the tubular hollow member (30) but somewhat thickened and is mounted in the interior (38) of the hollow member (24, 26) for axial displacement, and comprises an outflow duct (54) open to the interior (38) of the hollow member, and that the element is adapted to be controllably adjustable from the outside.

2. An outlet valve for a pressure fluid from a sphygmomanometer, having a chamber (66), the interior of which is connected to the inflow side (78) of the pressure medium, having a hollow member (64) with resilient walls which is situated at least partially in the chamber and is of tubular construction, open at one end and has an interior with a substantially uniform cross-section, wherein a slit (74) is provided in at least one of the walls of the hollow member (64) which are situated in the chamber (66) and the chamber is otherwise closed with a sealing action and wherein the interior of the hollow member (64) is connected to the outflow side of the pressure fluid, and having an element (72) influencing the opening of the slit, characterised in that the element is formed by a screw (72) which is disposed in a thread (68) in the wall of the chamber (66) and presses on the front end wall of the tubular hollow member (64), and the slits (74) extend parallel to the side walls of the hollow member (64), and that the element is adapted to be controllably adjustable from the outside.

3. An outlet valve as claimed in Claim 1, wherein the interior (38) of the resilient hollow member (30) is cylindrical in construction, characterised in that the expanding element (28, 44) is likewise cylindrical and is mounted (24, 26) in the chamber (2) so that it is displaced axially by turning a portion (50) projecting out of the chamber.

4. An outlet valve as claimed in one of the preceding Claims, characterised in that click-stop points are provided for the displacement travel of the element (28; 72).

**Revendications**

1. Soupape de décharge pour un fluide sous pression sortant d'un appareil de mesure de la pression sanguine avec une chambre (2) dont l'enceinte intérieure (10) est reliée au côté d'adduction (12) du fluide sous pression, avec une pièce creuse (30) se trouvant, au moins partiellement, dans la chambre et avec des parois élastiques, qui présente une forme tubulaire, est ouverte à une extrémité et a une enceinte intérieure de section essentiellement uniforme tandis qu'une fente (40, 42) est prévue dans au moins l'une des parois de la pièce creuse (30) se trouvant dans la chambre et que la chambre est fermée hermétiquement, d'autre part, et ou l'enceinte inférieure (38) de la pièce creuse est reliée au côté d'évacuation du fluide sous pression (54) et avec un élément (28) influençant l'ouverture de la fente, caractérisée en ce que l'élément se présente sous forme d'un écarteur (28) avec une section (44) correspondant à la pièce creuse tubulaire (30), mais avec une légère surépaisseur et disposée dans l'enceinte extérieure (38) de la pièce creuse (24, 26), avec possibilité de déplacement axial et qui présente un canal d'évacuation (54) ouvert vers l'enceinte intérieure (38) de la pièce creuse, et en ce que l'élément est conçu pour être réglé de l'extérieur.

2. Soupape de décharge pour un fluide sous pression provenant d'un appareil de mesure de la pression sanguine, avec une chambre (66) dont l'enceinte intérieure est reliée avec le côté d'adduction (78) du fluide sous pression, avec une pièce creuse (64) se trouvant, au moins partiellement, dans la chambre et avec des parois élastiques qui est constituée en forme de tube ouvert à une extrémité et avec une enceinte intérieure de section essentiellement uniforme où au moins une fente (74) est prévue dans les parois de la pièce creuse (64) se trouvant dans la chambre (66) et où la chambre est fermée hermétiquement pour le reste et où l'enceinte intérieure de la pièce creuse (64) est reliée avec le côté d'evacuation de fluide sous pression et avec un élément (72) influençant l'ouverture de la fente, caractérisée en ce que l'élément est constitué par une vis (72) placée dans un filetage (66) dans la paroi de la chambre (66), cette vis appuyant sur la paroi de fermeture de la pièce creuse tubulaire (64) et les fentes (74) s'étendant parallèlement aux parois latérales de la pièce creuse (64), et en ce que l'élément est disposé de manière à pouvoir être réglé de l'extérieur.

3. Soupape de décharge selon la revendication 1, dont la pièce creuse élastique (30) a son enceinte intèrieure (38) de forme cylindrique, caractérisée en ce que l'écarteur (28, 44) est également cylindrique et qu'il est disposé (24, 26) dans la chambre (2) en sorte qu'il peut se déplacer axialement par suite de la rotation d'une pièce (50) faisant saillie hors de la chambre.

4. Soupape de décharge selon l'une des revendications précédentes, caractérisée en ce que des points d'engrènement sont prévus sur le trajet de déplacement de l'élément (28, 72).

FIG.1

FIG.4

FIG.2

FIG.3

FIG.5

FIG.6

FIG.7